(19) Europäisches Patentamt
European Patent Office
Office européen des brevets

(11) Numéro de publication: **0 234 986**
**A1**

(12) **DEMANDE DE BREVET EUROPEEN**

(21) Numéro de dépôt: 87400130.8

(22) Date de dépôt: 20.01.87

(51) Int. Cl.³: C 08 L 93/04
C 09 F 1/04, G 03 C 1/72
C 07 C 69/753
//G03H1/18

(30) Priorité: 20.01.86 FR 8600697

(43) Date de publication de la demande: 02.09.87 Bulletin 87/36

(84) Etats contractants désignés: DE GB NL


(71) Demandeur: ETAT FRANCAIS représenté par le Ministre des PTT (Centre National d'Etudes des Télécommunications)
38-40 rue du Général Leclerc
F-92131 Issy-les-Moulineaux(FR)

(72) Inventeur: Moisan, Jean-Yves
21, rue du Dauphiné
F-22300 Lannion(FR)

(72) Inventeur: Lever, Roger
Poul Ar Marrant Plourin Les Morlaix
F-29210 Morlaix(FR)

(72) Inventeur: Daviaud, Raymond
6, rue Callixte Camelle
F-33400 Talence(FR)

(72) Inventeur: Servens, Christian
102, rue Villepreux village
F-33160 Saint-Aubin de Medoc(FR)

(74) Mandataire: Warcoin, Jacques et al,
Cabinet Régimbeau 26, avenue Kléber
F-75116 Paris(FR)


(54) **Compositions résiniques stables dans le temps et en température, esters résiniques autostabilisés, procédé de préparation et utilisation comme matériau photo-thermoplastique.**


(57) La présente invention concerne une composition résinique stable dans le temps et en température, caractérisée en ce qu'elle comprend au moins un acide résinique ou un dérivé d'acide résinique associé à moins un élément stabilisant du type capable de libérer un proton.

Elle concerne notamment les esters résiniques comportant un stabilisant greffé, et un procédé de préparation de ces esters.

Elle concerne enfin leur utilisation comme matériau photothermoplastique et comme stabilisant.


EP 0 234 986 A1

# COMPOSITIONS RESINIQUES STABLES DANS LE TEMPS ET EN TEMPERATURE, ESTERS RESINIQUES AUTOSTABILISES, PROCEDE DE PREPARATION ET UTILISATION COMME MATERIAU PHOTO-THERMOPLASTIQUE

La présente invention concerne une composition résinique stable dans le temps et en température comprenant un acide ou dérivé d'acide de type résinique associé à un stabilisant.

Ces compositions sont notamment utiles pour leur application comme matériau photo-thermoplastique.

Les matériaux photo-thermoplastiques sont intéressants pour le stockage de l'information, en particulier, pour l'enregistrement d'images effaçables, par exemple des hologrammes tels que des réseaux holographiques, dans le domaine de la commutation optique. L'information est enregistrée par exposition, par exemple à des rayons lasers puis développée par traitement thermique au point de ramollissement. Elle est effacée par chauffage à une température plus élevée.

Pour cette application, les composés utilisés doivent présenter d'une part un bon rendement de diffraction - le rendement maximum théorique est de 33,9 %-, et d'autre part, une bonne stabilité au cours des cycles écriture/lecture/effacement ou enregistrement/effacement.

Dans le domaine de la commutation optique, il est connu d'utiliser des dérivés de la colophane, notamment des esters abiétiques, qui ont de bons rendements de diffraction, mais qui supportent un nombre insuffisant de cycles.

Ces esters sont des produits de condensation obtenus par estérification des acides abiétiques hydrogénés ou perhydrogénés avec le glycérol ou le pentaérythritol.

Les forts rendements de diffraction obtenus pour ces produits semblent liés à la forte énergie d'activation de la viscosité : parfaitement amorphes, ils voient leur viscosité changer énormément avec la température.

Ce comportement rhéologique est particulièrement intéressant, en particulier pour les applications comme matériau photothermoplastique.

Ces produits industriels posent cependant un problème de pureté dû à la fabrication et à la dégradation pendant le stockage.

La présence d'impuretés accentue en effet les dégradations des composés actuellement utilisés. La dégradation thermique est vraisemblablement due à une réaction en chaîne conduisant à une cétone par l'intermédiaire d'un peroxyde. Ce processus peut être suivi par analyse spectrophotométrique dans le domaine infra-rouge, la teneur en hydroperoxyde permettant de définir un indice de dégradation ID.

L'essentiel de la dégradation des photothermoplastiques, lors de leur utilisation en commutation optique holographique, se produit lors de la charge électrique du composé par décharges corona. Ce processus de dégradation par décharge est moins connu que la dégradation thermique. Là aussi cependant, on a pu définir un indice de dégradation IDC. On n'observe pas toutefois en spectrophotométrie dans l'infra-rouge, la présence de radicaux hydroperoxydes observés lors des essais de dégradation uniquement thermique. Le processus semble donc différent.

La présente invention a pour objet des compositions résiniques présentant une stabilité accrue tant au stockage que lors de l'utilisation.

Dans le cas d'applications comme matériaux photothermoplastiques, la stabilité au cours des cycles enregistrement/effacement est particulièrement importante.

La stabilisation des compositions selon la présente invention repose à la fois sur l'utilisation de composés exempts d'impuretés et également sur l'utilisation de stabilisants.

C'est pourquoi la présente invention concerne une composition résinique stable dans le temps et en température, caractérisée en ce qu'elle comprend au moins un acide résinique ou un dérivé d'acide résinique associé à au moins un élément stabilisant du type capable de libérer un proton.

En particulier, les acides du type résinique, selon l'invention, sont des acides organiques à squelette tricyclique

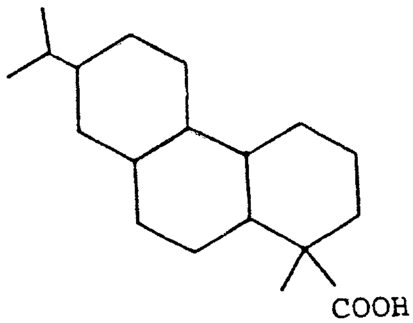

dont les cycles sont plus ou moins hydrogénés.

Parmi ces acides il faut citer l'acide abiétique saturé, insaturé ou partiellement insaturé.

En effet, l'acide abiétique,

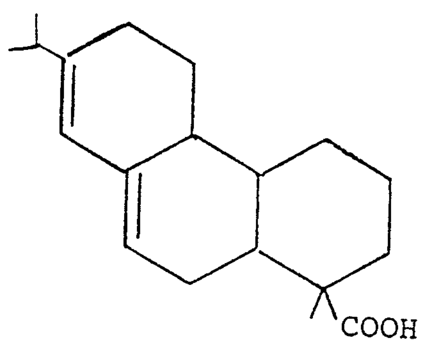

par une ou plusieurs hydrogénations ou deshydrogénations, conduit à d'autres acides du même type, comme l'acide déhydro-abiétique

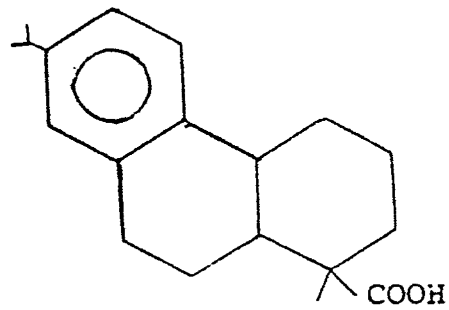

ou par per-hydrogénation à l'acide saturé de formule

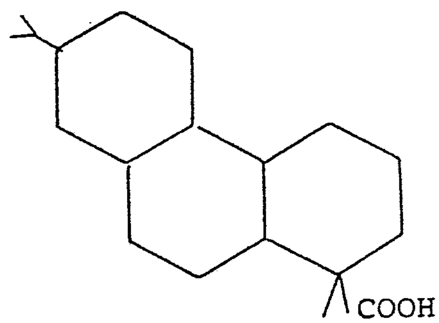

Les dérivés d'acide selon l'invention sont, comme on le verra, par la suite, en général des esters en particulier des esters de polyols ou d'autres alcools à fonctions non hydroxylées.

Les problèmes liés à la stabilité sont résolus selon l'invention par la présence de stabilisants associés aux acides définis ci-dessus ou leurs dérivés.

Ces éléments stabilisants peuvent se présenter sous diverses formes. L'élément stabilisant peut être un composé chimique portant un radical chimique stabilisant, ou bien l'élément stabilisant peut être un radical chimique stabilisant porté par l'acide résinique ou le dérivé d'acide résinique. Enfin, il est possible d'utiliser un acide résinique ou un dérivé d'acide résinique portant un radical

chimique stabilisant comme élément stabilisant d'un autre acide résinique ou dérivé d'acide résinique.

Parmi les radicaux chimiques stabilisants susceptibles de libérer un proton, il faut citer ceux comportant :

a) une fonction phénolique bloquée telle que :

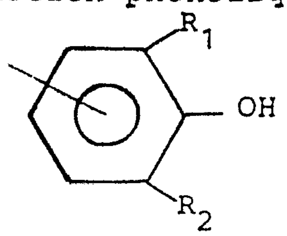

dans laquelle $R_1$ et $R_2$ sont des radicaux alkyles volumineux à fort encombrement stérique en $C_3$ à $C_{10}$, par exemple tertiobutyle,

b) une fonction amine à empêchement stérique telle que les fonctions tétraalkylpipéridine ou diarylamine notamment les tétra ($C_1$ à $C_3$)alkylpipéridine et la N-phénylnaphtyl-amine ou leurs dérivés.

Parmi les composés chimiques stabilisants correspondant à des fonctions phénoliques bloquées, il faut citer le 2,6-ditertiobutyl-4-hydroxyméthyl-phénol, d'autres phénols bloqués étant connus sous le nom d'Irganox®, Ionol®, Ionox®, Topanol® et similaires.

D'autre part, parmi les amines à encombrement stérique, il faut citer en particulier les N-phénylnaphtylamines, par exemple le Tinuvin® et similaires.

Selon un premier aspect de l'invention, les compositions sont réalisées par mélange du composé de type résinique et de stabilisant.

Comme composé de type résinique, il faut citer particulièrement les esters du type résinique, notamment les esters connus sous le nom de SE 10, Foral, par exemple.

De telles compositions comprennent outre l'ester résinique, un stabilisant du type phénolique ou amine à encombrement stérique, par exemple de l'Irganox® ou du Tinuvin®.

La Demanderesse a pu mettre en évidence l'efficacité de cette stabilisation comme cela apparaîtra dans les exemples. Ces compositions sont stables lors du stockage, ce qui présente un grand intérêt.

En effet, les produits non stabilisés, même après leur purification, doivent en général être utilisés dans les 48 heures.

Dans le cas des compositions selon l'invention, les tests de stabilité au stockage à température élevée d'une part, la loi de cinétique d'autre part, permettent de penser qu'à température ambiante, on peut les conserver pendant quelques mois.

De plus, ces compositions sont stables au cours de l'utilisation en commutation optique, où intervient une décharge corona à chaque enregistrement.

Ainsi, si avec un ester abiétique commercial, il est possible d'enregistrer des réseaux de diffraction ayant un rendement de diffraction entre 18 et 20 %, on a montré cependant que des rendements supérieurs ne sont obtenus qu'au prix d'une forte accélération de la dégradation.

Or, les mêmes rendements de diffraction sont obtenus avec des compositions selon l'invention comportant par exemple un ester abiétique stabilisé avec seulement jusqu'à environ 10 % de stabilisant du type phénolique bloqué, par exemple 2 % d'Irganox® 1035.

On a montré donc que les conditions physico-chimiques nécessaires pour l'enregistrement d'images holographiques étant remplies par un ester abiétique chargé de stabilisant, l'adjonction d'un stabilisant ne remet pas en cause l'usage du thermoplastique stabilisé en tant que matériau photo-thermoplastique.

Mais surtout le nombre de cycles enregistrement/effacement est fortement amélioré par la présence de stabilisants.

Donc le comportement dans le temps d'un ester abiétique -avant et pendant son utilisation- peut être considérablement amélioré outre par purification chimique du produit commercial, par addition d'un stabilisant de type phénolique bloqué ou amine à empêchement stérique.

L'adjonction à des esters abiétiques classiques d'un tel composé phénolique ou amine, cristallisable dans le domaine de températures utilisées, dans des proportions supérieures à 10 %, n'est cependant pas souhaitable du point de vue rhéologie du produit final.

La Demanderesse a alors résolu ce problème en plaçant le radical chimique stabilisant sur l'acide du type résinique. L'association de l'acide du type résinique et du stabilisant consiste ici en une fixation chimique.

Ces esters résolvent d'une part, le problème de la stabilité dans le temps et en température, de la stabilité lors des enregistrements/effacements, et d'autre part, des problèmes de rhéologie évoqués ci-dessus.

Ils sont donc particulièrement adaptés à l'application comme matériaux photo-thermoplastiques.

Les fonctions mises en oeuvre ont déjà été évoquées précédemment, la fixation pouvant s'effectuer par toute technique chimique, par exemple l'estérification de la fraction acide d'un acide résinique par un dérivé hydroxylé portant la fonction stabilisatrice.

Ainsi, la Demanderesse a synthétisé par exemple des esters de formule

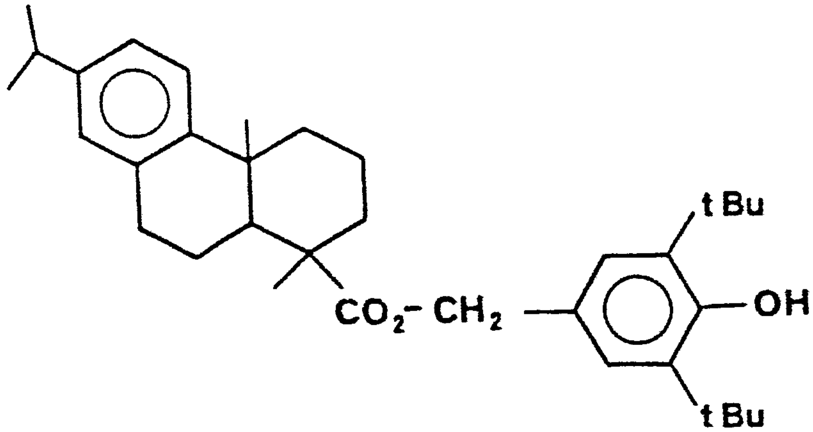

TC1

et

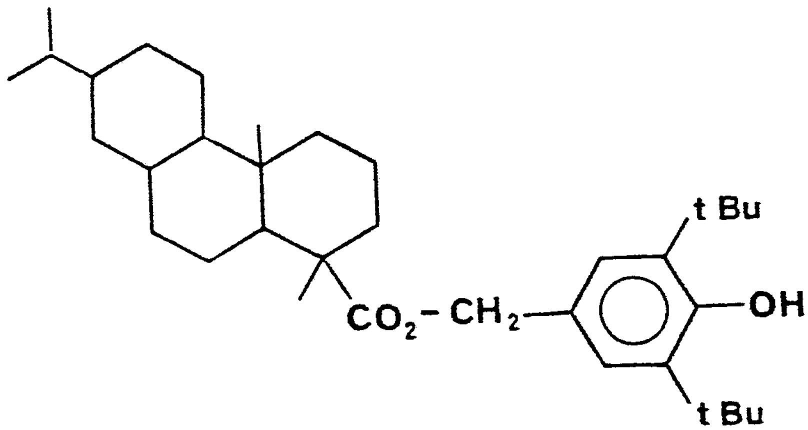

TCAB

Des composés du même type peuvent être proposés en utilisant d'autres acides résiniques et d'autres éléments stabilisants par exemple les amines à encombrement stérique portant une fonction hydroxy.

Ces composés résiniques portant une fonction stabilisante peuvent être utilisés comme agents stabilisants pour d'autres dérivés d'acide résinique ou bien être utilisés seuls à titre d'élements essentiels de la composition.

La Demanderesse a effectué des études comparatives de la viscosité des esters selon l'invention (TC1) par rapport à celles d'un ester abiétique commercial. L'énergie d'activation est du même ordre de grandeur. La Demanderesse

a d'autre part comparé la résistivité d'un ester abiétique commercial et de deux échantillons de pureté différente d'un ester du type résinique greffé selon l'invention (TC1). Elle a pu constater que l'évolution est sensiblement la même avec la température. Les conditions physico-chimiques nécessaires pour l'enregistrement d'images holographiques sont donc également remplies par un ester de type résinique modifié à l'aide d'un substituant portant la fonction stabilisant. Il s'agit donc de photothermoplastiques auto-stabilisés.

C'est pourquoi, la présente invention concerne l'utilisation de ces esters du type résinique à titre de matériau photo-thermoplastique. De bons rendements de diffraction ont été obtenus avec l'ester TC1 seul, bien que sa pureté n'ait été que de 75 %.

Enfin, ces esters du type résinique sont, comme cela a été indiqué, par nature des stabilisants, puisque leur structure chimique comporte, greffé, un substituant stabilisant. Ils peuvent donc être incorporés, et en toutes proportions, à des matériaux ou compositions photo-thermoplastiques à stabiliser.

Ainsi, on a montré que de bons rendements de diffractions sont obtenus en mélangeant un ester abiétique commercial avec un ester greffé selon l'invention à titre de stabilisant, par exemple 2 % de l'ester TC1 selon l'invention.

Ils peuvent également être utilisés comme stabilisants dans d'autres applications, dans le domaine des adhésifs hot melt ou des peintures thermiquement stables notamment.

C'est pourquoi, la présente invention concerne également l'utilisation des esters selon l'invention comme stabilisant.

L'invention concerne également un procédé de préparation d'un composé résinique comportant un radical chimique stabilisant caractérisé en ce que :

- on estérifie un acide résinique ou un dérivé réactif d'acide résinique avec un composé choisi parmi :
  . les amines à encombrement stérique hydroxylée et
  . les phénols bloqués comporant une fonction hydroxy non phénolique,

et en ce que
- on sépare l'ester obtenu.

Parmi les acides résiniques utilisables, il faut citer l'acide abiétique, déhydroabiétique et perhydroabiétique notamment sous forme d'halogénure, par exemple de chlorure d'acide.

Le phénol utilisé peut être par exemple le 2,6-ditertiobutyl-4-hydroxyméthylphénol.

Les conditions de l'estérification sont les conditions habituelles en particulier l'utilisation d'un agent fixant l'acide chlorhydrique tel que la triéthylamine.

Enfin, l'invention concerne un procédé de purification des esters d'acide résinique, en particulier avec des polyols, caractérisé en ce qu'on dissout ces esters dans l'heptane normal à une concentration comprise 0,5 g/l à 20 g/l puis la solution obtenue est filtrée pour éliminer les impuretés.

En particulier, des concentrations de 2 % à 7 % sont bien adaptées à cette purification.

Ce procédé de purification est suivi par une reprise des produits purifiés. On obtient ainsi des composés purifiés dont la stabilité est notablement augmentée.

D'autres caractéristiques et avantages de l'invention apparaîtront à la lecture des exemples ci-après et sur les figures annexées.

La figure 1 représente les résultats de résistance à la dégradation thermique (ID) en fonction du temps (h) pour l'ester abiétique SE 10 non stabilisé, à 110°C (1), stabilisé par 0,5 % de NPNA, à 100°C (2), et stabilisé par 0,5 % d'Irganox® 1035, à 110°C (3).

La figure 2 représente les résultats de résistance à la dégradation (IDC) en fonction du nombre de cycles pour

l'ester abiétique SE 10 non stabilisé (1), stabilisé par 0,5 % de Tinuvin® 770 (2) et par 0,5 % d'Irganox® 1035 (3), à 120°C.

EXEMPLE 1 :

Purification des esters

- 5 g d'ester sont dissous dans 1 litre d'heptane normal.

- La solution obtenue est filtrée sur filtre en fibres de verre permettant de retenir toutes les particules de dimension supérieure à 0,2 µm. La solution obtenue est légèrement colorée suivant l'ester, mais parfaitement limpide.

- La solution est évaporée à sec au rotavapor, c'est-à-dire sous vide obtenue à la trompe à eau et à température modérée (inférieure à 80° C).

- Le produit solide est repris dans un minimum d'éther éthylique, versé dans un bécher de PTFE. L'éther est évaporé sous chauffage, mais à l'abri des poussières. L'évaporation est terminée sous vide à 100°C. Le refroidissement du produit solide se fait sous vide primaire.

- Le produit obtenu est alors conservé au froid et utilisé en général dans les 48 heures.

EXEMPLE 2 :

Efficacité pendant le stockage

Cette efficacité a été mesurée en suivant l'évolution de l'indice de dégradation. Un film de quelques micromètres d'un ester abiétique connu, stabilisé est analysé régulièrement au cours d'une exposition à température élevée. La figure 1 qui correspond à l'analyse spectrophotométrique IR montre qu'en présence d'un phénolique bloqué (ici l'Irganox® 1035) ou d'une amine à empêchement stérique (ici la N-phénylnaphtyl-amine ou NPNA), les

cinétiques de dégradation sont considérablement ralenties par rapport à l'ester non stabilisé.

EXEMPLE 3 :

Synthèse de TC 1

Cet ester abiétique phénolé n'existe pas commercialement. La synthèse en a été entreprise. Toutefois, l'acide abiétique étant un mélange d'isomères (ceux-ci plus ou moins hydrogénés, dans ce cas un mélange d'acides hydrogénés ou perhydrogénés), la synthèse a été modelisée en prenant l'acide déhydroabiétique, cristallisable, et que donc l'on peut purifier suivant les méthodes habituelles de la chimie organique.

La synthèse a donc été réalisée en partant de l'acide déhydroabiétique.

On prépare d'abord un chlorure d'acide par traitement d'une solution benzénique de l'acide à l'aide d'un excès de chlorure de thionyle fraichement distillé. Après un reflux de deux heures, le benzène est évaporé.

L'ester est préparé en solution benzénique avec un excès de triéthylamine et de 2,6-ditertiobutyl-4-hydroxyméthyl-phénol par rapport au chlorure d'acide. Le mélange réactionnel est agité pendant 20 heures à la température ambiante. Le chlorhydrate de triéthylamine est éliminé par filtration et le benzène évaporé. On reprend à l'éther, on lave par de la soude à 10 %, puis à l'eau et on sèche la phase éthérée. Le résidu est repris à l'hexane ; la solution est filtrée, pour éliminer le produit phénolique restant.

EXEMPLE 4 :

Le TCAB est synthétisé suivant une procédure identique à celle de l'exemple 3 à partir de l'acide abiétique perhydrogéné, produit disponible commercialement.

EXEMPLE 5 :

Stabilité en commutation optique

Lors de l'utilisation des thermoplastique en commutation optique, l'essentiel de la dégradation se produit lorsque le matériau est chargé électriquement par effet corona.

Le cycle modelisé comprend une charge (1 s) suivi d'un repos (5 s) dans un cycle proche de celui réalisé en commutation optique pour des essais dits de "vieillissement". La dégradation est chiffrée par un indice IDC mesuré par spectrophotométrie dans l'infra-rouge (décroissance de l'absorbance caractéristique des liaisons C-H de l'ester abiétique). La figure 2 montre à l'évidence l'effet bénéfique de la présence d'un phénolique bloqué (ici Irganox© 1035) ou d'une amine à empêchement stérique (ici Tinuvin© 770), par mesure d'IDC pour des esters stabilisés (2 et 3) par rapport à l'ester non stabilisé (1).

Le critère choisi pour évaluer la stabilité au cours des cycles est le nombre de cycles effectués avec un rendement supérieur à 5 %, c'est-à-dire le nombre de cycles effectués lorsque cette limite de 5 % est atteinte.

L'ester abiétique commercial (SE 10) permet d'effectuer entre 3 000 et 5 000 cycles, dans les conditions opératoires exposées ci-dessus.

Entre 6 000 et 9 000 cycles peuvent être effectués lorsque la purification du même ester abiétique est convenablement conduite.

De 10 000 à 13 000 cycles sont possibles avec une stabilisation due à environ 2 % d'Irganox© 1035 dans l'ester abiétique purifié.

## REVENDICATIONS

1. Composition résinique stable dans le temps et en température, caractérisée en ce qu'elle comprend au moins un acide résinique ou un dérivé d'acide résinique purifié associé à au moins un élément stabilisant du type capable de libérer un proton.

2. Composition selon la revendication 1, caractérisée en ce que le dérivé de l'acide résinique est choisi parmi les esters de l'acide abiétique saturé, insaturé ou partiellement insaturé.

3. Composition selon la revendication 2, caractérisée en ce que l'ester est un ester de polyol.

4. Composition selon la revendication 3, caractérisée en ce que l'ester est un ester de glycérol ou de pentaérythritol.

5. Composition selon l'une des revendications 1 à 4, caractérisée en ce que l'élément stabilisant est un composé chimique portant un radical chimique stabilisant.

6. Composition selon la revendication 1, caractérisée en ce que l'élément stabilisant est un radical chimique stabilisant porté par l'acide résinique ou le dérivé d'acide résinique.

7. Composition selon l'une des revendications 5 et 6, caractérisée en ce que le radical chimique stabilisant est une fonction phénolique bloquée ou une fonction amine à empêchement stérique.

8. Composition selon la revendication 7, caractérisée en ce que la fonction phénolique bloquée est du type

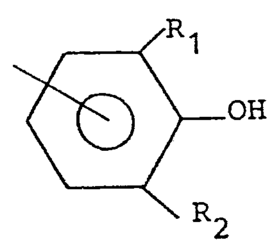

où $R_1$ et $R_2$ sont des radicaux alkyles volumineux.

9. Composition selon la revendication 8, caractérisée en ce que la fonction phénolique est bloquée en 2,6 par des tertiobutyl.

10. Composition selon les revendications 5 et 6, caractérisée en ce que le radical chimique stabilisant est un radical amino à encombrement stérique.

11. Composition selon la revendication 10, caractérisée en ce que le radical amino à encombrement stérique est choisi parmi les radicaux de type tétraalkylpipéridine ou diarylamine.

12. Composition selon les revendications 1 et 5 à 11, caractérisée en ce que le radical chimique stabilisant est fixé sur l'acide résinique ou le dérivé d'acide résinique par l'intermédiaire d'une fonction ester.

13. Composition selon la revendication 1, caractérisée en ce qu'elle comporte au moins un ester de l'acide abiétique déhydroabiétique ou perhydroabiétique avec une amine à encombrement stérique hydroxylée ou un phénol bloqué comportant une fonction hydroxy non phénolique.

14. Composition selon la revendication 1, caractérisée en ce qu'elle comporte au moins un ester de l'acide abiétique, déhydroabiétique ou perhydroabiétique et une amine à encombrement stérique ou un phénol bloqué.

15. Procédé de préparation d'un composé résinique comportant un radical chimique stabilisant caractérisé en ce que :

- on estérifie un acide résinique ou un dérivé réactif d'acide résinique avec un composé choisi parmi :
  - . les amines à encombrement stérique hydroxylée et
  - . les phénols bloqués comportant une fonction hydroxy non phénolique,

et en ce que

- on sépare l'ester obtenu.

16. Procédé selon la revendication 15, caractérisé en ce que l'acide résinique est un dérivé de l'acide abiétique, déhydroabiétique ou tétrahydroabiétique sous forme d'halogénure et en ce que le phénol utilisé est le 2,6-ditertiobutyl-4-hydroxyméthylphénol.

17. Procédé de purification des esters d'acides résiniques notamment des esters d'acides abiétiques et analogues entrant dans la composition selon la revendication 1, caractérisé en ce qu'on dissout ces esters dans l'heptane normal à une concentration comprise entre 0,5 g et 20 g/l puis la solution obtenue est filtrée pour éliminer les impuretés.

18. Procédé de purification selon la revendication 17, caractérisé en ce que la concentration est comprise entre 2 g/l et 7 g/l.

19. Application des compositions selon l'une des revendications 1 à 14 pour la réalisation d'éléments photothermoplastiques.

1/2

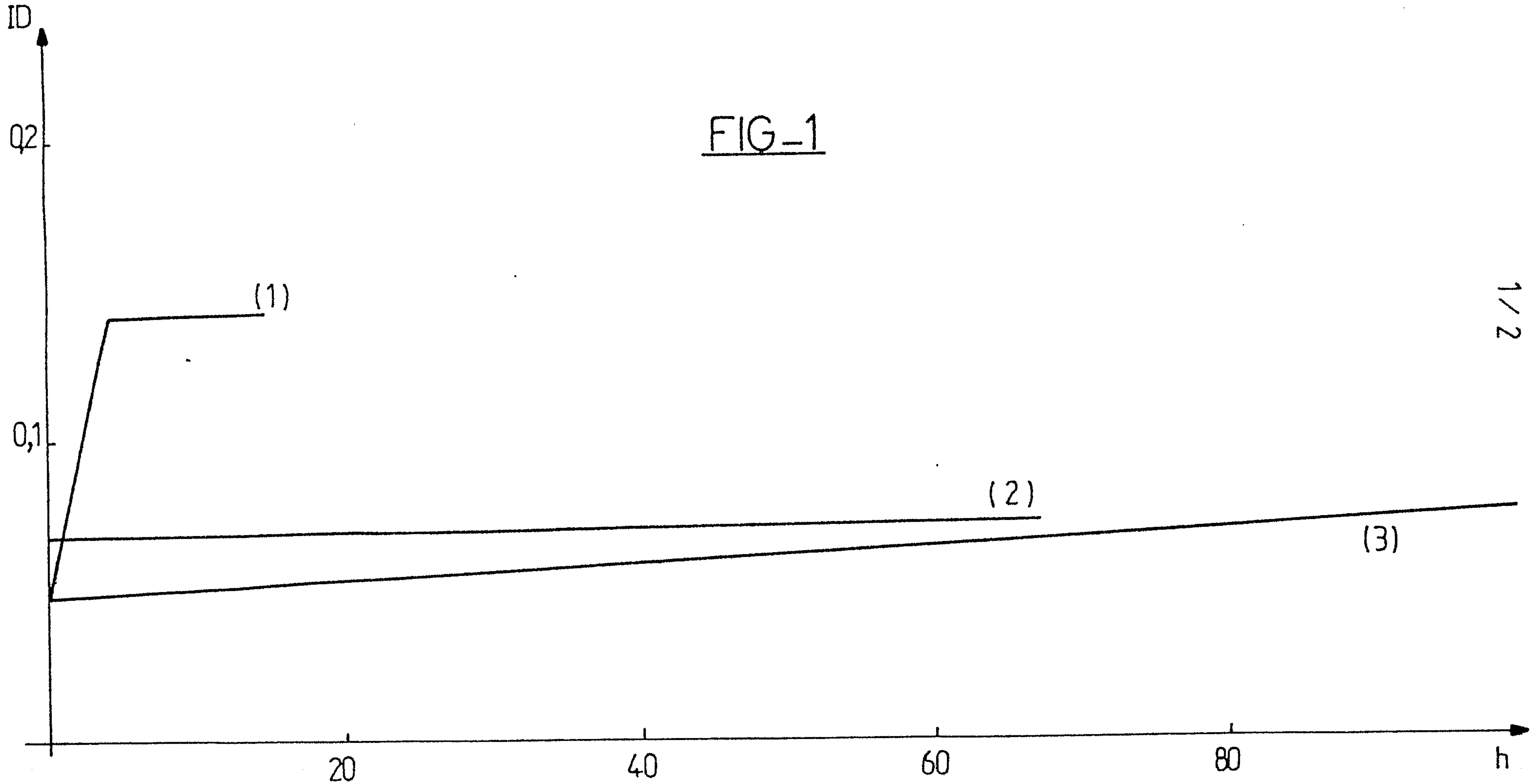


FIG-1

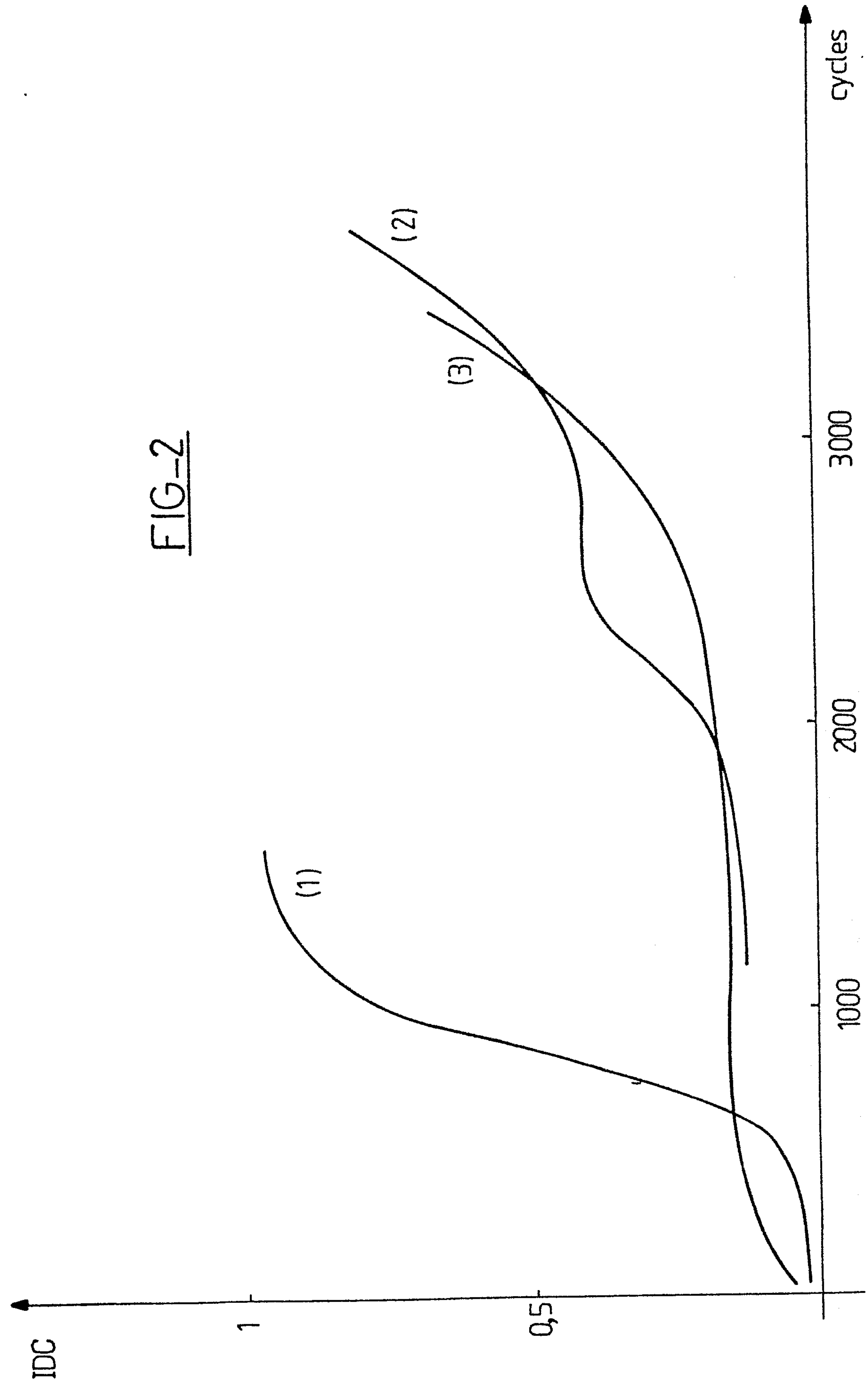


FIG-2

Office européen des brevets

# RAPPORT DE RECHERCHE EUROPEENNE

Numéro de la demande 0234986

EP 87 40 0130

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | US-A-2 294 723 (A.C. DRESHFIELD)<br>* Whole document *<br>--- | 1-5,7, 11,14 | C 08 L 93/04<br>C 09 F 1/04<br>G 03 C 1/72<br>C 07 C 69/753//<br>G 03 H 1/18 |
| X | CHEMICAL ABSTRACTS, vol. 80, 1974, page 96, résumé no. 28628m, Columbus, Ohio, US; & JP-A-73 52 806 (SHINTO PAINTS CO., LTD) 25.07.1973<br>--- | 1-5,7-11,14 | |
| X | CHEMICAL ABSTRACTS, vol. 97, 1982, page 48, résumé no. 56881h, Columbus, Ohio, US; & PO-A-109 696 (INSTYTUT CHEMII PRZEMYSLOWEJ) 10.03.1982<br>--- | 1-5,7-11,14 | |
| X | CHEMICAL ABSTRACTS, vol. 79, 1973, page 32, résumé no. 67231a, Columbus, Ohio, US; M. TOYAMA et al.: "Oxidative aging of tackifiers", & NIPPON SECCHAKU KYOKAI SHI 1973, 9(3), 120-6<br>--- | 1 | DOMAINES TECHNIQUES RECHERCHES (Int Cl 4)<br>C 08 L 93<br>C 09 F 1<br>G 03 C 1<br>C 07 C 69<br>G 03 C 5<br>B 41 M 5<br>G 11 B 7 |
| X | CHEMICAL ABSTRACTS, vol. 94, 1981, page 90, résumé no. 48968q, Columbus, Ohio, US; & SU-A-767 160 (ALL-UNION SCIENTIFIC RESEARCH INSTITUTE OF COMPLEX PRINTING PROBLEMS, KIEV) 30.09.1980<br>--- -/- | 1-5,7-9,14 | |

Le présent rapport de recherche a été établi pour toutes les revendications

| Lieu de la recherche | Date d'achèvement de la recherche | Examinateur |
|---|---|---|
| LA HAYE | 27-04-1987 | PHILOSOPH L.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

# RAPPORT DE RECHERCHE EUROPEENNE

Numéro de la demande 0234986

EP 87 40 0130



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | CHEMICAL ABSTRACTS, vol. 98, 1983, page 55, résumé no. 35748a, Columbus, Ohio, US; & CS-A-194 893 (B. BUCEK) 30.07.1982 --- | 1-5,7-9,14 | |
| A | CHEMICAL ABSTRACTS, vol. 73, 1970, page 369, résumé no. 135931g, Columbus, Ohio, US; & SA-A-69 06 125 (STALEY, A.E., MANUFG. CO.) 04.03.1970 --- | 19 | |
| A | CHEMICAL ABSTRACTS, vol. 75, 1971, page 555, résumé no. 13594v, Columbus, Ohio, US; SA-A-70 CO 384 (STALEY A.E., MANUFG. CO.) 04.09.1970 --- | 19 | |
| A | CHEMICAL ABSTRACTS, vol. 86, 1977, page 379, résumé no. 49166y, Columbus, Ohio, US; N.G. KUVSHINSKII et al.: "Two kinds of phase holograms in photothermoplastic materials", & J. SIGNALAUFZEICHNUNGSMATERIALIEN 1976, 4(4), 243-9 --- | 19 | DOMAINES TECHNIQUES RECHERCHES (Int. Cl.4) |
| A | US-A-3 562 243 (P.H. ALDRICH) ----- | 17 | |

Le présent rapport de recherche a été établi pour toutes les revendications

| Lieu de la recherche | Date d'achèvement de la recherche | Examinateur |
|---|---|---|
| LA HAYE | 27-04-1987 | PHILOSOPH L.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82